(19) Europäisches Patentamt
European Patent Office
Office européen des brevets

(11) EP 0 653 201 B1

(12) **EUROPÄISCHE PATENTSCHRIFT**

(45) Veröffentlichungstag und Bekanntmachung des Hinweises auf die Patenterteilung:
**08.07.1998 Patentblatt 1998/28**

(51) Int. Cl.[6]: **A61K 7/09**, C07C 323/58

(21) Anmeldenummer: **94113108.8**

(22) Anmeldetag: **23.08.1994**

(54) **Mittel zur dauerhaften Haarverformung sowie neue haarkeratinreduzierende Cysteinglycerinester**

Agent for permanent hair waving and new hair keratine reducing cysteine glycerol esters

Agent pour la mise en plis permanente des cheveux ainsi que des nouveaux esters de la cystéine et la glycérine réducteurs de la kératine des cheveux

(84) Benannte Vertragsstaaten:
**DE ES FR GB IT**

(30) Priorität: **28.10.1993 DE 4336838**

(43) Veröffentlichungstag der Anmeldung:
**17.05.1995 Patentblatt 1995/20**

(73) Patentinhaber:
**Wella Aktiengesellschaft**
**64274 Darmstadt (DE)**

(72) Erfinder:
- **Lang, Günther, Dr.**
  **D-64354 Reinheim (DE)**
- **Braun, Hans-Jürgen, Dr.**
  **CH-3182 Ueberstorf (CH)**
- **Maresch, Gerhard**
  **D-64295 Darmstadt (DE)**

(56) Entgegenhaltungen:
**EP-A- 0 518 137**
**DE-A- 2 658 424**
**US-A- 4 992 267**

EP 0 653 201 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Mittel zur dauerhaften Haarverformung sowie neue haarkeratinreduzierende Cysteinglycerinester.

Für eine schonende dauerhafte Verformung von geschädigtem, insbesondere gebleichtem oder gefärbtem Haar werden bevorzugt schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Dem Vorteil einer haarschonenderen Verformungsbehandlung durch schwach saure bis neutrale Verformungsmittel stehen jedoch eine Reihe von Nachteilen gegenüber. So besitzen diese Mittel eine im Vergleich zu mildalkalischen Verformungsmitteln auf Thioglykolatbasis geringere Wellwirksamkeit. Aus diesem Grunde ist für die Erzielung einer ausreichenden Umformung die Zufuhr von Wärme, eine Verlängerung der Einwirkungszeiten auf 20 bis 60 Minuten sowie die Verwendung von vergleichsweise dünnen Wicklern erforderlich. Eine Verwendung dieser Verformungsmittel für normales, nicht geschädigtes Naturhaar erscheint aufgrund der auch bei Wärmezufuhr erforderlichen langen Einwirkungszeiten von über 30 Minuten nicht sinnvoll, so daß die Anwendung von schwach sauer bis neutral eingestellten Verformungsmitteln bisher in der Regel auf vorgeschädigtes, leicht verformbares Haar beschränkt blieb.

Ein weiterer erheblicher Nachteil von sauren Haarverformungsmitteln ist die schlechte Augen- und Hautverträglichkeit sowie die sensibilisierende Wirkung der Thioglykolsäureester.

Trotz einer Vielzahl von Versuchen konnte bisher die sensibilisierende Wirkung von sauren Haarverformungsmitteln nicht entscheidend verringert werden

Als Alternative wurden daher mildalkalische (pH = 7,1 bis 9) Haarverformungsmittel vorgeschlagen, die als keratinreduzierenden Wirkstoff Cystein oder dessen Salze enthalten.

Diese Haarverformungsmittel weisen jedoch ebenfalls eine Reihe von Nachteilen auf. So besitzt Cystein nur eine schwache Verformungswirksamkeit sowie eine geringe Stabilität. Wenn cysteinhaltige Verformungsmittel auf das Haar aufgebracht werden, wird das Cystein durch den Luftsauerstoff schnell zu in Wasser schwer löslichem Cystin oxidiert, welches sich in Form eines störenden, schwer entfernbaren weißen Belages auf dem Haar ablagert.

Aus der DE-A-26 58 424 sind Kalt-Dauerwellmittel bekannt, welche als Hauptbestandteil einen niederen Alkylester des Cysteins, Z. B. Cysteinmethyl-, ethyl- oder -propylester, enthalten. In der US-A-4 992 267 sind Haarentkräuselungsmittel auf der Basis von Alkalihydroxyden beschrieben, die zusätzlich geringe Mengen einer keratinsulfid-reduzierenden Verbindung mit Mercaptogruppe, unter anderem niedere Alkylester des Cysteins wie den Methyl-, Ethyl- und Propylester, enthalten.

Der Erfindung liegt daher die Aufgabe zugrunde, ein Mittel zur dauerhaften Haarverformung zur Verfügung zu stellen, das sowohl im sauren als auch schwach alkalischen Bereich (pH-Wert = 4,5 bis 9,0) eine schonende und gleichmäßige Umformung ermöglicht, kein oder aber nur ein geringes Allergiepotential aufweist und bei dem darüber hinaus keine störenden Ablagerungen oder Beläge auf dem Haar entstehen.

Überraschenderweise wurde nunmehr gefunden, daß mit Mitteln zur dauerhaften Verformung von Haaren auf der Basis eines Glycerinesters des Cysteins eine gleichmäßige Umformung sowohl von geschädigtem als auch ungeschädigtem Haar möglich ist, ohne daß hierbei die bei den bisher für die Haarverformung verwendeten haarkeratinreduzierenden Esterverbindungen häufig beobachteten allergischen Hautreaktionen auftreten oder die bei cysteinhaltigen Dauerverformungsmitteln beobachteten störenden Beläge auf dem Haar entstehen.

Gegenstand der vorliegenden Erfindung ist daher ein Mittel zur dauerhaften Verformung von Haaren auf der Basis eines keratinreduzierenden Wirkstoffes, welches als keratinreduzierenden Wirkstoff einen Glycerinester des Cysteins enthält.

Als Glycerinester des Cysteins (nachfolgend Cysteinglycerinester genannt) können sowohl die Mono- als auch die Di- und Triglyceride des Cysteins verwendet werden, wobei der Monoglycerinester des Cysteins besonders bevorzugt ist.

Zwar ist es möglich den Cysteinglycerinester gemeinsam mit anderen keratinreduzierenden Wirkstoffen - wie zum Beispiel Thioglykolsäure, Thiomilchsäure, 3-Hydroxy-2-mercaptopropionsäure, Cysteamin und Cysteaminderivaten oder Cystein und Cysteinderivaten - zu verwenden, jedoch ist die Verwendung des Cysteinglycerinesters als alleinigem keratinreduzierenden Wirkstoff (das heißt ohne Zusatz anderer keratinreduzierender Wirkstoffe) besonders bevorzugt.

Der Cysteinglycerinester wird in dem gebrauchsfertigen erfindungsgemäßen Mittel zur dauerhaften Haarverformung in einer Menge von 4 bis 30 Gewichtsprozent, vorzugsweise in einer Menge von 8 bis 28 Gewichtsprozent, eingesetzt.

Das gebrauchsfertige erfindungsgemäße Verformungsmittel besitzt einen pH-Wert von 4,5 bis 9,0, vorzugsweise von 6,0 bis 8,5.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle; Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Da Carbonsäureester in wäßrigem Medium nur für begrenzte Zeit haltbar sind und, insbesondere im alkalischen Bereich, leicht hydrolysieren können, wird der Cysteinglycerinester vorzugsweise in wasserfreier Form abgepackt und das Verformungsmittel erst unmittelbar vor Gebrauch durch Vermischen der den Cysteinglycerinester enthaltenden Komponente mit einer oder mehreren weiteren Komponenten hergestellt.

Je nach Konfektionierung kann das erfindungsgemäße Mittel hierbei in Form eines Zwei- oder Dreikomponenten-Präparates vorliegen.

So ist das erfindungsgemäße Mittel zum Beispiel durch Vermischen zweier Komponenten erhältlich, von denen die erste Komponente das oder die Alkalisierungsmittel, beispielsweise ein Alkalicarbonat, Ammoniumcarbonat, Alkalihydrogencarbonat oder Ammoniumhydrogencarbonat, sowie die vorstehend genannten kosmetischen Zusatzstoffe und Wasser enthält, während die zweite, wasserfreie Komponente den Cysteinglycerinester enthält.

Ebenfalls ist es möglich, das erfindungsgemäße Mittel in Form eines Dreikomponenten-Präparates abzupacken, wobei eine Komponente einen Teil der vorstehend genannten kosmetischen Zusatzstoffe sowie Wasser, eine zweite, wasserfreie Komponente den Cysteinglycerinester und die dritte Komponente Parfümöle, Lösungsvermittler und Pflegestoffe in wäßriger Lösung oder wasserfreier Form enthält.

Bei allen Ausführungsformen des erfindungsgemäßen Mittels können die vorstehend genannten kosmetischen Zusatzstoffe sowohl in der wäßrigen als auch in der/den nichtwäßrigen Komponente(n) enthalten sein.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze.

Die dauerhafte Verformung der Haare wird in der üblichen Weise durchgeführt, indem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet.

Hierbei kann das Haar zunächst mit einem Shampoo gewaschen und danach mit Wasser gespült werden. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die in den erfindungsgemäßen Mitteln zur Verformung der Haare enthaltenden Cysteinglycerinester können in üblicher Weise durch Veresterung von Cystein in Gegenwart eines sauren Katalysators, beispielsweise Schwefelsäure, Salzsäure oder Benzolsulfonsäure, mit Glycerin, vorzugsweise unter Wärmeeinwirkung, erhalten werden.

Es ist jedoch auch möglich, die Cysteinmonoglycerinester durch Alkylierung des Dinatriumsalzes des Cystins mit 3-Chlor- oder 3-Brom-1,2-propandiol und anschließende Reduktion des erhaltenen Cystin-diglycerylesters herzustellen.

Die vorgenannten Cysteinglycerinester besitzen eine hohe Wellwirksamkeit bei sauren bis leicht alkalischen pH-Werten, sind nahezu geruchsneutral und leicht wasserlöslich und weisen eine hervorragende physiologische Verträglichkeit sowie eine gute Stabilität in Wasser auf.

Die Cysteinglycerinester sind aus der Literatur noch nicht bekannt.

Ein weiterer Gegenstand der vorliegenden Anmeldung sind daher die neuen Mono-, Di- und Triglyceride des Cysteins ("Cysteinglycerinester"), von denen die entsprechenden Monoglyceride des Cysteins ("Cysteinmonoglycerinester")
2-Amino-3-mercaptopropionsäure-(2',3'-dihydroxypropyl) ester (I) und
2-Amino-3-mercaptopropionsäure (1',3'-dihydroxyisopropyl)ester (II)
- alleine oder in Kombination, besonders bevorzugt sind

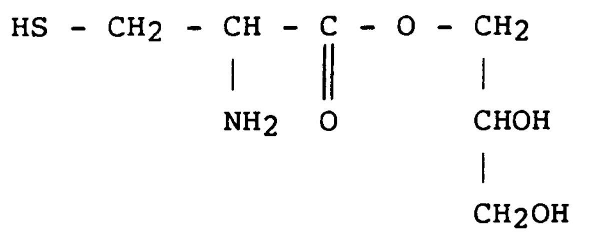

(I)

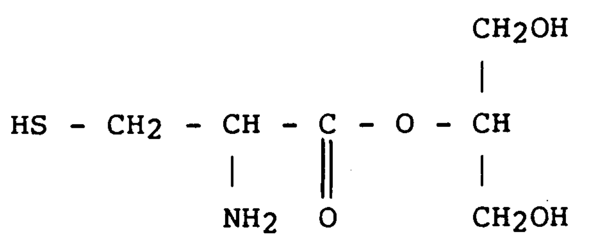

(II)

Die nachfolgenden Beispiele sollen den Gegenstand der Erfindung näher erläutern.

**Beispiele für Haarverformungsmittel**

**Beispiel 1: Zweikomponenten-Haarverformungsmittel**

| | | |
|---|---|---|
| Komponente 1: | 3,0 g | Harnstoff |
| | 1,0 g | Octylphenol, mit 20 Mol Ethylenoxid oxethyliert |
| | 0,8 g | Ammoniak (25-prozentige wäßrige Lösung) |
| | 0,5 g | Parfümöl |
| | 0,3 g | Ammoniumhydrogencarbonat |
| | 94,4 g | Wasser, vollentsalzt |
| | 100,0 g | |

| | | |
|---|---|---|
| Komponente 2: | 70,0 g | Cysteinmonoglycerinester gemäß Beispiel 4 |
| | 30,0 g | Glycerin |
| | 100,0 g | |

Vor dem Gebrauch werden 70 Gramm der Komponente 1 und 30 Gramm der Komponente 2 zu einem Haarverformungsmittel mit einem pH-Wert von 6,3 vermischt.

Leicht vorgeschädigtes Haar wird mit einem Shampoo gewaschen und mit Wasser gründlich gespült. Anschließend wird das frottierte Haar auf Wickler mit einem Durchmesser von 8 Millimetern gewickelt, mit dem Haarverformungsmittel gleichmäßig befeuchtet und mit einer Plastikhaube abgedeckt. Nach einer Einwirkzeit von 15 Minuten bei 40°C wird die Plastikhaube entfernt, das Haar mit Wasser gespült und mit 100 Gramm einer 3-prozentigen Hydrogenperoxidlösung oxidativ nachbehandelt.

Nach Entfernung der Wickler werden die Haare erneut mit Wasser gespült und danach getrocknet.

Als Ergebnis dieser Behandlung wird eine gleichmäßige, natürlich wirkende Umformung der Haare vom Haaransatz bis zu den Haarspitzen erhalten.

**Beispiel 2: Zweikomponenten-Dauerverformungsmittel**

| | | |
|---|---|---|
| Komponente 1: | 2,0 g | Dipropylenglykolmonomethylether |
| | 1,0 g | Ammoniak (25-prozentige wäßrige Lösung |
| | 0,3 g | Kokosfettalkohol, mit 10 Mol Ethylenoxid oxethyliert |
| | 0,3 g | Parfümöl |
| | 0,2 g | Diammoniumdithiodiglykolat |
| | 96,2 g | Wasser, vollentsalzt |
| | 100,0 g | |

| | | |
|---|---|---|
| Komponente 2: | 60,0 g | Cysteinmonoglycerinester gemäß Beispiel 4 |
| | 30,0 g | Glycerin |
| | 10,0 g | N-Acetylcysteamin |
| | 100,0 g | |

80 Gramm der Komponente 1 werden mit 40 Gramm der Komponente 2 zu einem gebrauchsfertigen Haarverformungsmittel vom pH 6,5 vermischt.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 20 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so umgeformte Haar besitzt eine über die gesamte Haarlänge gleichmäßige Krause, die mit der durch Behandlung mit mildalkalischen Haarverformungsmittel erzielten Krause vergleichbar ist.

**Beispiel 3 Dreikomponenten-Dauerverformungsmittel**

| | | |
|---|---|---|
| Komponente 1: | 1,9 g | 1,2-Propylenglykol |
| | 1,0 g | Glycerindiacetat |
| | 0,8 g | Kokosfettalkohol, mit 10 Mol Ethylenoxid oxethyliert |
| | 0,3 g | Parfümöl |
| Komponente 2: | 4,0 g | Harnstoff |
| | 1,5 g | Ammoniak (25-prozentige wäßrige Lösung) |
| | 1,2 g | Ammoniumhydrogencarbonat |
| | 89,3 g | Wasser |
| Komponente 1 +2: | 100,0 g | |

| | | |
|---|---|---|
| Komponente 3: | 80,0 g | Cysteinmononglycerinester gemäß Beispiel 4 |
| | 20,0 g | Glycerin |
| | 100,0 g | |

Komponente 1 wird unmittelbar vor der Anwendung in Komponente 2 gelöst. Anschließend werden 70 Gramm dieser Lösung mit 30 Gramm der Komponente 3 vermischt. Der pH-Wert des gebrauchsfertigen Dauerverformungsmittels beträgt 6,8.

Ein Haar von ungleichmäßiger Haarqualität wird gewaschen, mit einem Handtuch frottiert und auf Wickler mit einem Durchmesser von 6 Milimetern gewickelt. Anschließend wird das Haar mit dem vorstehend beschriebenen gebrauchsfertigen Dauerverformungsmittel gleichmäßig durchfeuchtet.

Nach einer Einwirkungszeit von 15 Minuten bei 40°C wird das Haar mit Wasser gespült, zur Wasserwelle gelegt und anschließend getrocknet.

Als Ergebnis dieser Behandlung wird ein gleichmäßig verformtes Haar mit hoher Sprungkraft und Elastizität erhalten.

**Herstellungsbeispiel**

**Beispiel 4:** 2-Amino-3-mercaptopropionsäure-(2',3'-dihydroxypropyl)ester

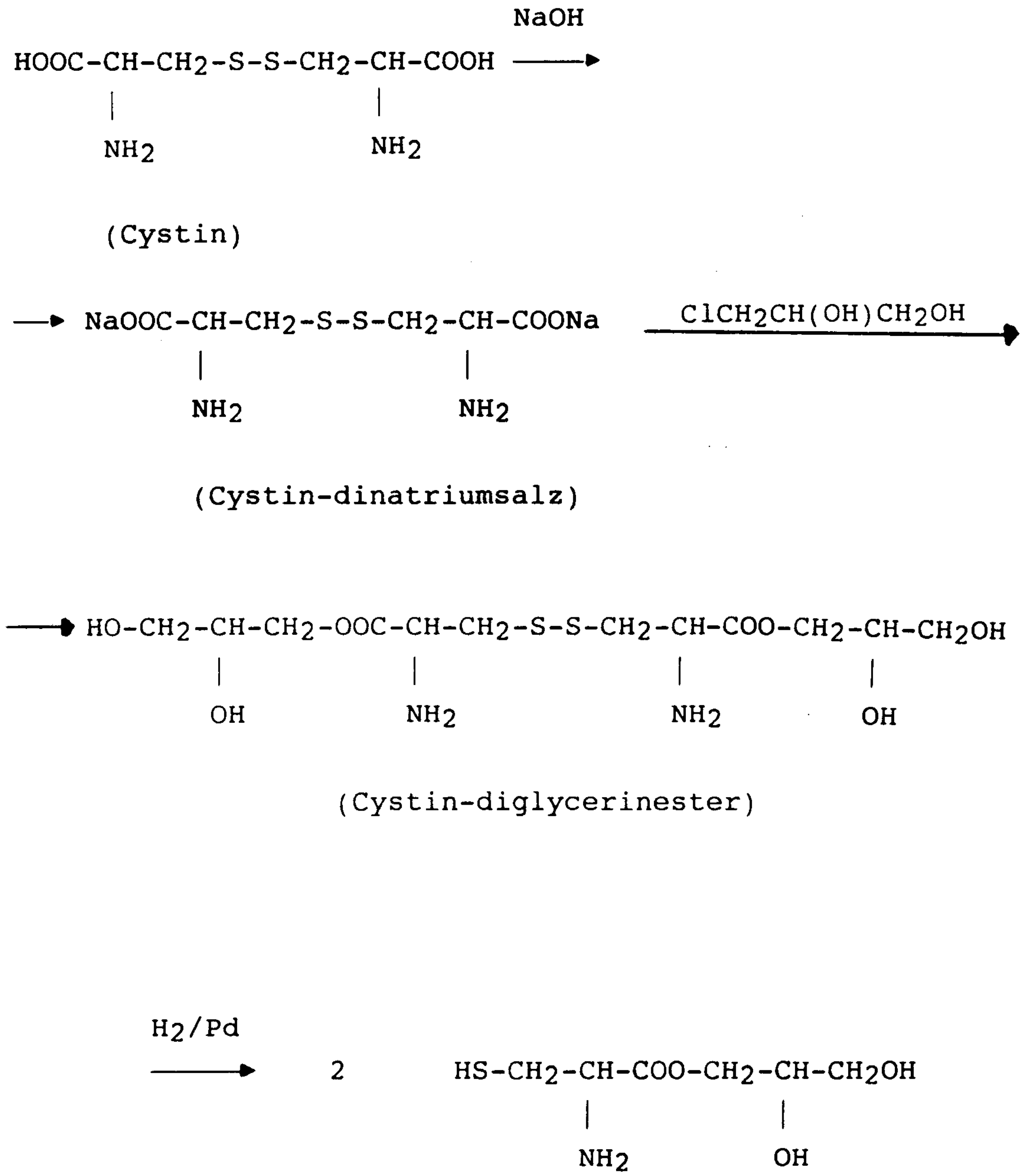


(Cystin)

(Cystin-dinatriumsalz)

(Cystin-diglycerinester)

25,6 g (0,09 Mol) trockenes Cystin-dinatriumsalz, hergestellt aus Cystin und der für die Salzbildung erforderlichen Menge Natriumhydroxid, werden zusammen mit 31 g (0,2 mol) 3-Brom-1,2-propandiol in 50 ml Glykoldimethylether 3 Stunden lang unter Rückfluß erwärmt. Das Lösungsmittel wird anschließend abdestilliert, der Rückstand in verdünnter Salzsäure aufgenommen und sodann in Gegenwart eines Palladium/Aktivkohle-Katalysators hydriert. Nach Aufnahme der berechneten Menge an Wasserstoff wird der Katalysator abfiltriert, das Filtrat neutralisiert und anschließend mit Methylenchlorid extrahiert. Nach dem Trocknen der Methylenchloridphase über Magnesiumsulfat und Abdestillieren des Methylenchlorids erhält man 14 g (≙ 39,8 % der Theorie) eines öligen Produktes (≙ 2-Amino-3-mercaptopropionsäure-(2',3'-dihydroxypropyl)ester), welches ohne weitere Reinigung in Dauerverformungsmitteln eingesetzt werden

kann.

**Beispiel 5:** 2-Amino-3-mercaptopropionsäure-(2',3'-dihydroxypropyl)ester

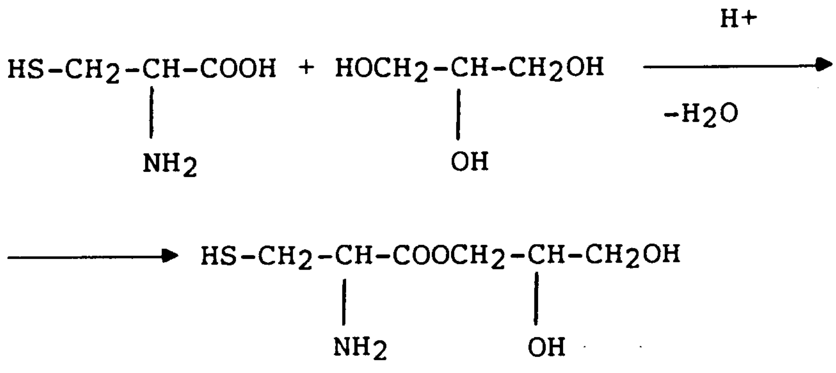


12,1 g (0,1 Mol) L-Cystein werden gemeinsam mit 100 ml Glycerin und 50 ml 3-normaler Salzsäure 3 Stunden lang auf 90° G erwärmt.

Anschließend wird das bei der Reaktion entstandene Wasser bei 30 mbar abdestilliert (Destillationsdauer: 3 Stunden).

Der Destillationsrückstand enthält gemäß dem Dünnschichtchromatogramm (Stationäre Phase: Kieselgel 60; Laufmittel: Methanol/Wasser/Pyridin 80/20/4; rf-Wert (Cysteinmonoglycerinester)=0,75) kein Cystein. Der erhaltene 2-Amino-3-mercaptopropionsäure-(2',3'-dihydroxypropyl)ester kann ohne weitere Reinigung in Dauerverformungsmiteln verwendet werden.

Massenspektrum (70eV): m/e = 196 ($M^{+}\bullet$), 122,93,75,57

Alle in der vorliegenden Anmeldung enthaltenen Prozentangaben stellen, soweit nicht anders angegeben, Gewichtsprozent dar.

**Patentansprüche**

1. Mittel zur dauerhaften Verformung von Haaren auf der Basis eines keratinreduzierenden Wirkstoffes, dadurch gekennzeichnet, daß es als keratinreduzierenden Wirkstoff einen Glycerinester des Cysteins enthält.

2. Mittel nach Anspruch 1, dadurch gekennzeichnet, daß der Glycerinester des Cysteins der Monoglycerinester des Cysteins ist.

3. Mittel nach Anspruch 1 oder 2, dadurch gekennzeichnet daß es als alleinigen keratinreduzierenden Wirkstoff einen Glycerinester des Cysteins enthält.

4. Mittel nach einem der Ansprüche 1 bis 3, dadurch gekennzeichnet, daß der Glycerinester des Cysteins in dem gebrauchsfertigen Mittel in einer Menge von 4 bis 30 Gewichtsprozent enthalten ist.

5. Mittel nach einem der Ansprüche 1 bis 4, dadurch gekennzeichnet, daß der pH-Wert des gebrauchsfertigen Mittels 4,5 bis 9,0 beträgt.

6. Mittel nach einem der Ansprüche 1 bis 5, dadurch gekennzeichnet, daß es vor der Anwendung durch Vermischen von zwei oder drei Komponenten, wobei die den Glycerinester des Cysteins enthaltende Komponente wasserfrei ist, hergestellt wird.

7. Cysteinmonoglycerinester

8. Cysteindiglycerinester

9. Cysteintriglycerinester

**10.** 2-Amino-3-mercapto-propionsäure-(2',3'-dihydroxypropyl)ester

**11.** 2-Amino-3-mercapto-propionsäure-(1',3'-dihydroxyisopropyl)ester

## Claims

**1.** Agent for the permanent shaping of hair based on a keratin-reducing active substance, characterised in that it contains a glycerol ester of cysteine as the keratin-reducing active substance.

**2.** Agent according to Claim 1, characterised in that the glycerol ester of cysteine is the monoglycerol ester of cysteine.

**3.** Agent according to Claim 1 or 2, characterised in that it contains a glycerol ester of cysteine as the sole keratin-reducing active substance.

**4.** Agent according to any one of Claims 1 to 3, characterised in that the amount of the glycerol ester of cysteine contained in the ready-for-use agent is from 4 to 30% by weight.

**5.** Agent according to any one of Claims 1 to 4, characterised in that the pH of the ready-for-use agent is from 4.5 to 9.0.

**6.** Agent according to any one of Claims 1 to 5, characterised in that it is prepared prior to use by mixing two or three components, the component containing the glycerol ester of cystein being anhydrous.

**7.** Cystein monoglycerol ester.

**8.** Cystein diglycerol ester.

**9.** Cystein triglycerol ester.

**10.** 2-Amino-3-mercaptopropionic acid (2',3'-dihydroxypropyl)ester.

**11.** 2-Amino-3-mercaptopropionic acid (1',3'-dihydroxyisopropyl)ester.

## Revendications

**1.** Produit de mise en plis permanente des cheveux à base d'une substance active réduisant la kératine, caractérisé en ce qu'elle contient comme substance active réduisant la kératine un glycérinester de la cystéine.

**2.** Produit selon la revendication 1, caractérisé en ce que le glycérinester de la cystéine est le monoglycérinester de la cystéine.

**3.** Produit selon la revendication 1 ou 2, caractérisé en ce qu'il contient comme seule substance active réduisant la kératine, un glycérinester de la cystéine.

**4.** Produit selon l'une des revendications 1 à 3, caractérisé en ce que le glycérinester de la cystéine est contenu, dans le produit prêt à l'utilisation, en une quantité comprise entre 4 et 30 % en poids.

**5.** Procède sel on l'une des revendications 1 à 4, caractérisé en ce que le pH du produit près à l'utilisation est de 4,5 à 9,0.

**6.** Procédé sel on l'une des revendications 1 à 5, caractérisé en ce qu'il est préparé avant application par mélange de 2 ou 3 composants, les composants contenant le glycérinester de cystéine étant exempts d'eau.

**7.** Monoglycérinester de la cystéine.

**8.** Diglycérinester de la cystéine.

**9.** Triglycérinester de la cystéine.

**10.** 2-Amino-3-acide mercaptopropionique-(2',3'-dihydroxypropyl)ester.

**11.** 2-Amino-3-acide mercaptopropionique-(1',3'-dihydroxyisopropyl)ester.